# NEUE EUROPÄISCHE PATENTSCHRIFT

(11) **EP 1 997 875 B2**
(45) Veröffentlichungstag und Bekanntmachung des Hinweises auf die Entscheidung über den Einspruch: **25.01.2017**
(45) Hinweis auf die Patenterteilung: 04.08.2010
(21) Anmeldenummer: 08156915.4
(22) Anmeldetag: 26.05.2008
(51) Int. Cl.: C12M 1/107

(54) **Biogasanlage zur Erzeugung von Biogas aus Biomasse sowie Verfahren zum Betreiben der Biogasanlage**
Biogas facility for creating biogas from biomass and method for operating the biogas facility
Installation biologique de production de biogaz à partir de biomasse et procédé de fonctionnement d'une installation de biogaz

(30) Priorität: 29.05.2007 DE 102007024911
(43) Veröffentlichungstag der Anmeldung: 03.12.2008
(73) Patentinhaber: Bekon Energy Technologies GmbH & Co. KG, 85774 Unterföhring (DE)
(72) Erfinder: Lutz, Peter, 81927, München (DE)
(74) Vertreter: Schober, Mirko

(56) Entgegenhaltungen:
- EP-A- 1 681 274
- DE-U1- 20 319 847

## Beschreibung

Die Erfindung betrifft eine Biogasanlage zur Erzeugung von Biogas aus Biomasse mit wenigstens einem Fermenter nach Anspruch 1 sowie ein Verfahren zum Abschalten eines Fermenters nach Anspruch 10 und ein Verfahren zum Anfahren eines Fermenters nach Anspruch 14.

Die so genannte "Trockenfermentation" erlaubt es, schüttfähige Biomassen aus der Landwirtschaft, aus Bioabfällen und kommunalen Pflegeflächen zu methanisieren, ohne die Materialien in ein pumpfähiges, flüssiges Substrat zu überführen. Es können Biomassen mit bis zu 50% Trockensubstanzanteil vergoren werden. Dieses Trockenfermentations-Verfahren ist beispielsweise in der EP 0 934 998 beschrieben.

Bei der "trockenen" Vergärung wird das zu vergärende Material nicht in eine flüssige Phase eingerührt, wie das zum Beispiel bei der Flüssigvergärung von Bioabfällen der Fall ist. Stattdessen wird das in den Fermenter eingebrachte Gärsubstrat ständig feucht gehalten, indem das Perkolat am Fermenterboden abgezogen und über der Biomasse wieder versprüht wird. So werden optimale Lebensbedingungen für die Bakterien erreicht. Bei der Rezirkulation des Perkolats kann zusätzlich die Temperatur reguliert werden, und es besteht die Möglichkeit, Zusatzstoffe für eine Prozessoptimierung zuzugeben.

Aus der WO 02/06439 ist ein Bioreaktor bzw. ein Fermenter in Form einer Fartiggarage bekannt, der nach dem Prinzip der Trockenfermentation im sogenannten Batch-Verfahren betrieben wird. Hierbei wird nach einer Animpfung mit bereits vergorenem Material wird das Gärsubstrat mit Radladern in den Fermenter gefüllt. Der garagenförmig aufgebaute Gärbehälter wird mit einem gasdichten Tor verschlossen. Die Biomasse wird unter Luftabschluss vergoren, dabei erfolgt keine weitere Durchmischung und es wird kein zusätzliches Material zugeführt. Das aus dem Gärgut sickernde Perkolat wird über eine Drainagerinne abgezogen, in einem Tank zwischengespeichert und zur Befeuchtung wieder über dem Gärsubstrat versprüht. Der Gärprozess findet im mesophilen Temperaturbereich bei 34-37° C statt, die Temperierung erfolgt mittels einer Boden- und Wandheizung.

Das entstehende Biogas kann in einem Blockheizkraftwerk zur Gewinnung von Strom und Wärme genutzt werden. Damit immer genug Biogas für das Blockheizkraftwerk zur Verfügung steht, werden in der Trockenfermentationsanlage mehrere Gärbehälter zeitlich versetzt betrieben. Am Ende der Verweilzeit wird der Fermenterraum vollständig entleert und dann neu befüllt. Das vergorene Substrat wird einer Nachkompostierung zugeführt, so dass ein konventionellen Komposten vergleichbarer organischer Dünger entsteht.

Bedingt durch den Batch-Betrieb müssen die einzelnen Fermenter von Zeit zu Zeit abgeschaltet werden, d. h. die Biogasproduktion muss gestoppt werden, die vergorene Biomasse muss aus dem jeweiligen Fermenter entnommen und frische Biomasse muss in den Fermenter eingefüllt werden und die Biogasproduktion muss wieder aufgenommen werden. Hierbei muss aus sicherheitstechnischen verhindert werden, dass während des Ent- und Beladens der einzelnen Fermenter ein explosives Biogas/Luft-Gemisch entsteht.

Hierzu ist es aus der EP 1 301 583 B1 bekannt, einen laufenden Fermenter im Falle von Explosionsgefahr, d. h. Luft ist in den Fermenter eingedrungen, mit kohlendioxidhaltigem Abgas aus dem mit Biogas betriebenen Blockheizkraftwerk zu fluten. Das Dokument EP 1681274 A beschreibt einen Fermenter, der mit Kohlendioxidhaltigem Gas und mit Luft gespült werden kann.

Es ist daher Aufgabe der vorliegenden Erfindung ausgehend von einer Biogasanlage nach EP 1 301 583 B oder EP 1681274 A das Entladen von verbrauchter Biomasse aus und das Beladen des Fermenters mit frischer Biomasse sicher zu gestalten.

Die Lösung dieser Aufgabe erfolgt durch die Merkmale der Ansprüche 1, 10 und 14.

Die Biogasanlage nach Anspruch 1 umfasst die notwendigen Komponenten um ein sicheres Abschalten und Entladen und ebenso ein sicheres Anfahren eines Fermenters zu ermöglichen.

Durch die Maßnahmen nach Anspruch 10 wird die Biogasproduktion und -verwertung auch während des Abschaltens und Spülens mit kohlendioxidhaltigem Abgas solange wie möglich aufrecht erhalten, d. h. das Biogas/Abgas-Gemisch des abzuschaltenden Fermenters wird solange weiter dem Biogasverbraucher zugeführt, bis Qualität diese Gemisches unter einen vorbestimmten Grad absinkt. Erst wenn die Methankonzentration im Biogasauslass unter einen oberen Grenzwert absinkt, wird die zum Biogasverbraucher führende Biogasleitung von dem Biogasauslass getrennt. Danach wird das nur noch wenig Methan enthaltende Biogas/Abgas-Gemisch über einen Abluftkamin abgeführt. Dies erfolgt solange, bis die Methankonzentration auf einen unteren Grenzwert abgesunken ist, bei dem nahezu kein Methan mehr in dem Biogas/Abgas-Gemisch enthalten ist. Danach wird der abzuschaltende Fermenter anstelle mit kohlendioxidhaltigem Abgas mit Frischluft gespült und das Abgas/Biogas/Frischluft-Gemisch wird solange über den Abluftkamin abgeführt, bis die Kohlendioxidkonzentration in dem Abgas/Biogas/Frischluft-Gemisch auf einen ersten Grenzwert abgesunken ist. Erst dann wird der Fermenter geöffnet, um die verbrauchte Biomasse zu entladen und um den Fermenter erneut mit frischer Biomasse zu beladen. Durch die vorausgehenden Spülvorgänge mit Abgas und Frischluft ist das Öffnen und Ent- und Beladen des Fermenters ohne Gefahr für das Bedienungspersonal möglich.

Gemäß einer bevorzugten Ausgestaltung der Erfindung nach Anspruch 11 wird das Biogas/Abgas-Gemisch bei Erreichen des oberen Grenzwertes der Methankonzentration nicht über den Abluftkamin an die Umwelt abgegeben, sondern einer Abgasfackel zugeführt und dort abgefackelt, Gegebenfalls kann die Abgasfackel mit zusätzlichem Brennstoff versorgt werden, so dass in jedem Fall eine Verbrennung statt findet. Das Abfackeln des Biogas/Abgas-Gemisches erfolgt solange, bis die Methankonzentration in dem Biogas/Abgas-Gemisch einen mittleren Grenzwert unterschreitet, der zwischen dem oberen und dem unteren Grenzwert liegt.

Gemäß einer bevorzugten Ausgestaltung der Erfindung nach Anspruch 12 wird während des Ent- und Beladens des abgeschalteten Biofermenters über die offene Be- und Entladeöffnung Frischluft angesaugt und das angesaugte Gasgemisch wird über den Spülgaseinlass oder über den Biogasauslass dem Abluftkamin zugeführt. Alternativ kann auch ein spezieller Frischluftabsauganschluss in dem Fermenter vorgesehen werden.

Durch die vorteilhafte Ausgestaltung der Erfindung nach Anspruch 13 wird sichergestellt, dass während des Ent- und Beladens des Fermenters kontinuierlich Frischluft angesaugt wird

Durch das erfindungsgemäße Verfahren zum (Wieder-)Anfahren des abgeschalteten Fermenters nach Anspruch 14 und 15 wird sicher verhindert, dass beim Anfahren ein explosives Biogas/Luft-Gemisch entsteht.

Das Aufschalten dies wieder angefahrenen Fermenters auf die Biogasleitung erfolgt bei einem vierten Grenzwert der Methankonzentration, der gleich dem oberen Grenzwert ist - Anspruch 16.

Das Abgas zur Spülung des abzuschaltenden Fermenters wird beispielsweise von einer Verbrennungskraftmaschine bereit gestellt - Anspruch 17.

Gemäß einer bevorzugten Ausführungsform der Erfindung nach Anspruch 18 wird das kohlendioxidhaltige Abgas aus einer dem wenigstens einen Fermenter nach geschalteten Biogasaufbereitungseinrichtung bereitgestellt.

Die übrigen Unteransprüche beziehen sich auf vorteilhafte Ausgestaltungen der Erfindung.

Weitere Einzelheiten, Merkmale und Vorteile der Erfindung zeigt die nachfolgende Beschreibung beispielhafter Ausführungsformen anhand der Zeichnungen:
Es zeigt:
   Fig. 1 bis 7 schematische Darstellungen verschiedener Betriebszustände während des Abschaltens und während des (Wieder-) Anfahrens eines Fermenters;
   Fig. 8 eine schematische Darstellung einer zweiten Ausführungsform der Erfindung mit einem Fermenter; und
   Fig. 9 bis 15 schematische Darstellungen verschiedener Betriebszustände einer Biogasanlage mit drei Fermentern während des Abschaltens und während des (Wieder-) Anfahrens eines Fermenters

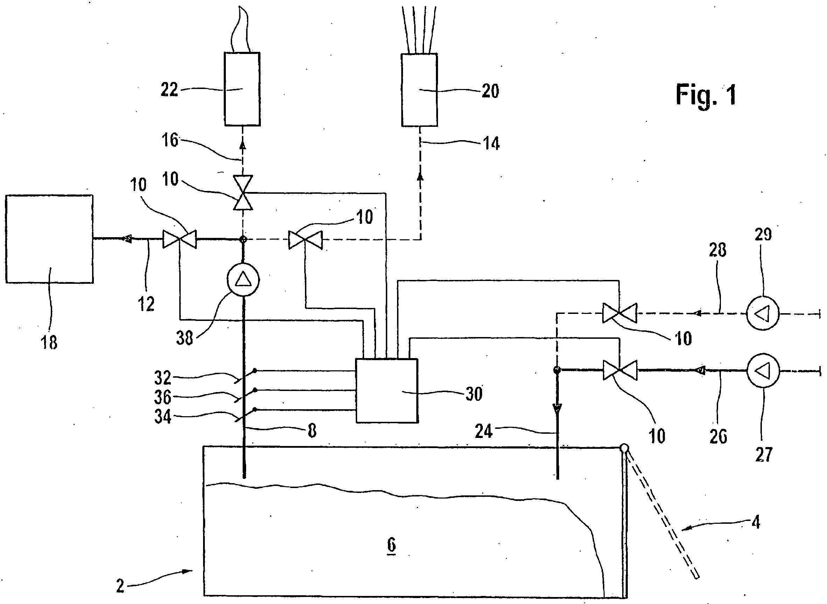

Die Figuren 1 bis 7 zeigen eine erste Ausführungsform einer Biogasanlage gemäß der vorliegenden Erfindung mit einem einzelnen Fermenter 2. Der Fermenter 2 ist quaderförmig und besitzt in etwa den Aufbau einer Fertiggarage. Über eine Be- und Entladeöffnung 4, die sich über eine der Stirnseiten des quaderförmigen Fermenters 2 erstreckt, lässt sich Biomasse 6 mittels eines Radladers in den Fermenter 2 einfüllen und wieder entnehmen. Hinsichtlich des genauen Aufbaus des Fermenters 2 wird auf die WO 02/06439 verwiesen.

Der Fermenter 2 umfasst weiter einen Biogasauslass 8, der über Ventile 10 mit einer Biogasleitung 12, einer ersten Biogas/Abgas-Leitung 14 und einer zweiten Biogas/Abgas-Leitung 16 verbindbar sind. Die Biogasleitung 12 führt zu einem Blockheizkraftwerk 18 als Biogasverwertungseinrichtung. Die erste Biogas/Abgas-Leitung 14 führt zu einem Abgaskamin 20. Die zweite Biogas/Abgas-Leitung 16 führt zu eine Abgasfackel 22, Weiter umfasst der Fermenter 2 einen Spülgaseinlass 24, der über Ventile 10 mit einer Abgasleitung 26 oder einer Frischluftleitung 28 verbindbar ist. In der Abgasleitung 26 ist ein Abgasgebläse 27 angeordnet, mittels dem Abgas in den Fermenter 2 gepumpt werden kann. In der Frischluftleitung 28 ist ein Frischluftgebläse 29 zum Ansaugen von Frischluft aus der Umgebung angeordnet. Über die Abgasleitung 26 wird kohlendioxidhaltiges Abgas als Spülgas und über die Frischluftleitung 28 wird Frischluft in den Fermenter 2 geführt.

Die Ventile 10 sind mit einer Steuereinrichtung 30 verbunden und werden durch die Steuereinrichtung 30 geöffnet oder geschlossen. Die Steuereinrichtung 30 ist auch mit einem ersten Messfühler 32 verbunden, der im Biogasauslass 8 angeordnet ist und die Methankonzentration in dem jeweiligen Gasgemisch erfasst. Die Steuereinrichtung 30 ist weiter mit einem zweiten Messfühler 34 verbunden, der ebenfalls im Biogasauslass 8 angeordnet ist und die Kohlendioxidkonzentration in dem jeweiligen Gasgemisch erfasst. Die Steuereinrichtung 30 ist auch mit einem dritten Messfühler 36 verbunden, der im Biogasauslass 8 angeordnet ist und den Gasvolumenstrom in dem Biogasauslass erfasst. Über ein in dem Biogasauslass angeordnetes Gebläse 38 kann der Abzug von Gas aus dem Fermenter 2 gegebenenfalls unterstützt werden.

In den Figuren 1 bis 7 sind verschieden Phasen des Abschaltens und Anfahrens des Fermenters 2 dargestellt, wobei aktive Leitungen und Stellungen von Komponenten durchgezogen dargestellt sind, während nichtaktive bzw. abgesperrte Leitungen und Stellungen von Komponenten strichliert dargestellt sind.

Fig. 1 zeigt die erste Phase des Abschaltens des Fermenters 2 bei der kohlendioxidhaltiges Abgas über die Abgasleitung 26 und den Spülgaseinlass 24 in das Innere des Fermenters 2 gepumpt wird. Der Biogasauslass 8 ist nach wie vor mit der Biogasleitung 12 verbunden, so dass das Biogas/Abgas-Gemisch weiter dem BHKW 18 zugeführt wird.

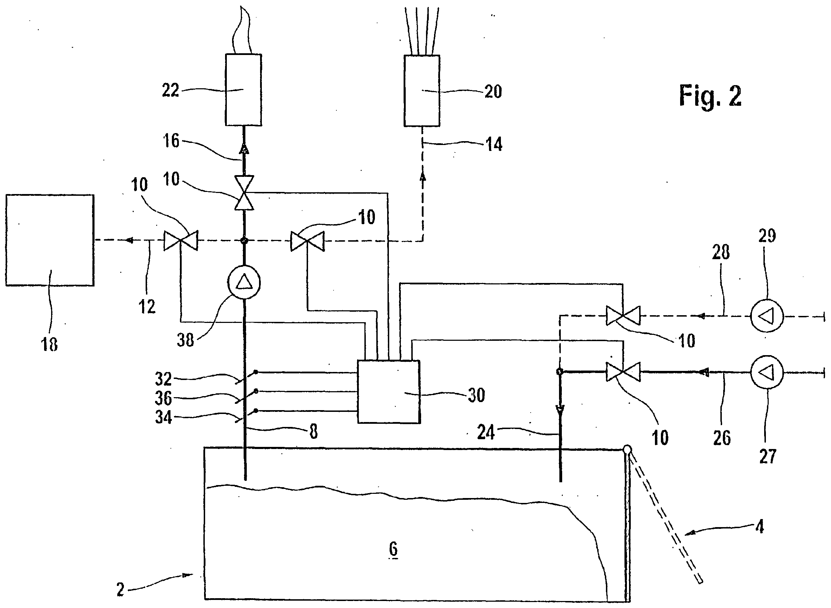

Erst wenn die durch den ersten Messfühler 32 in dem Biogasauslass 8 erfasste Methankonzentration unter einen oberen Grenzwert abgesunken ist, wird das Ventil 10 in der Biogasleitung 12 durch die Steuereinrichtung 30 geschlossen und das Ventil 10 in der zweiten Biogas/Abgas-Leitung 16 wird geöffnet, wie dies in Fig. 2 dargestellt ist. In dieser zweiten Phase des Abschaltens des Fermenters 2 wird das Biogas/Abgas-Gemisch in der Abgasfackel 22 verbrannt. Gegebenenfalls kann dieser Verbrennungsprozess durch Zugabe von zusätzlichem Brennstoff unterstützt werden.

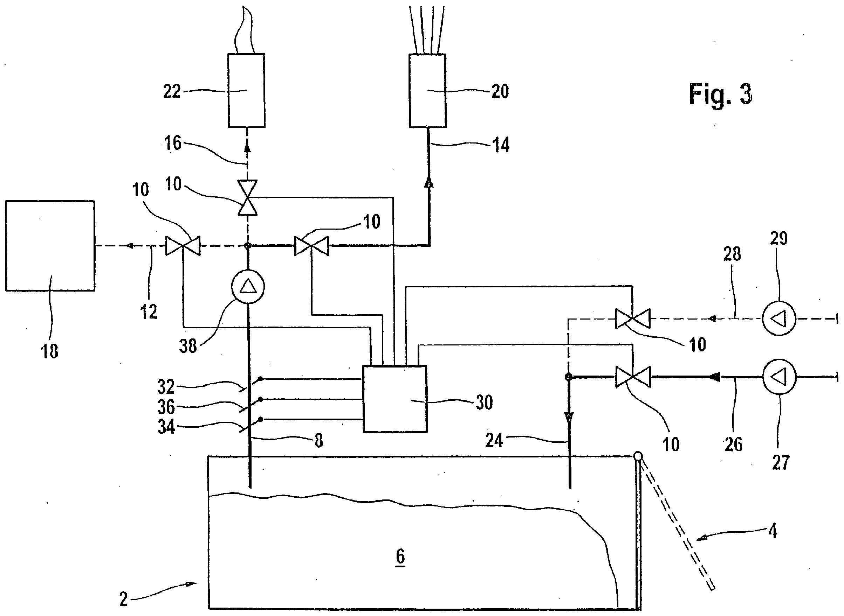

Wenn die durch den ersten Messfühler 32 in dem Biogasauslass 8 erfasste Methankonzentration unter einen mittleren Grenzwert abgesunken ist, wird das Ventil 10 in der zweiten Biogas/Abgas-Leitung 16 durch die Steuereinrichtung 30 geschlossen und das Ventil 10 in der ersten Biogas/Abgas-Leitung 14 wird geöffnet, wie dies in Fig. 3 dargestellt ist. In dieser dritten Phase des Abschaltens des Fermenters 2 wird das Biogas/Abgas-Gemisch über den Abgaskamin 20 an die Umgebung abgegeben.

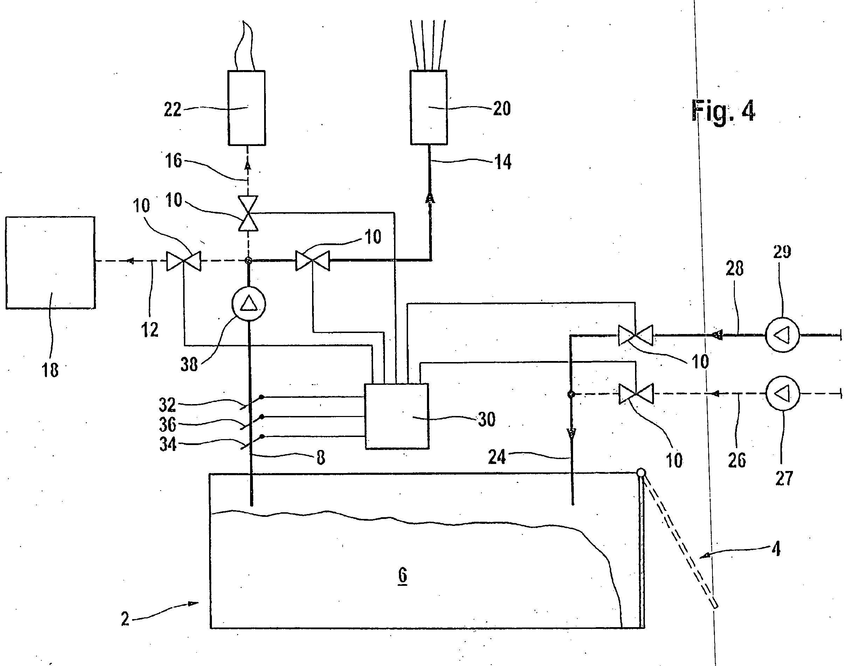

Wenn die durch den ersten Messfühler 32 in dem Biogasauslass 8 erfasste Methankonzentration unter einen unteren Grenzwert abgesunken ist, wird das Ventil 10 in der Abgasleitung 26 durch die Steuereinrichtung 30 geschlossen und das Ventil 10 in der Frischluftleitung 28 wird geöffnet, wie dies in Fig. 4 dargestellt ist. In dieser vierten Phase des Abschaltens des Fermenters 2 wird über die Frischluftleitung 28 und den Spülgaseinlass 24 Frischluft in den Fermenter 2 gepumpt. Das Abgas/Luft-Gemisch wird weiter über den Biogasauslass 8 und die erste Biogas/Abgas-Leitung 14 in dem Abgaskamin 20 an die Umgebung abgegeben.

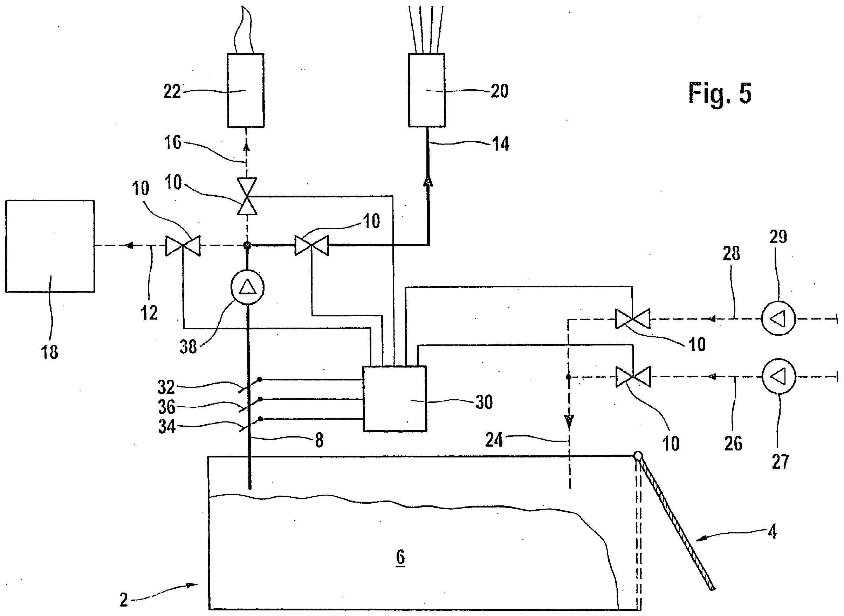

Wenn die durch den zweiten Messfühler 34 in dem Biogasauslass 8 erfasste Kohlendioxidkonzentration unter einen ersten Grenzwert abgesunken ist, wird das Ventil 10 in der Frischluftleitung 28 durch die Steuereinrichtung 30 geschlossen und die Be- und Entladeöffnung 4 geöffnet, wie dies in Fig. 5 dargestellt ist. Gleichzeitig wird über das Gebläse 38 Frischluft über die offene Be- und Entladeöffnung angesaugt und über den Abgaskamin 20 an die Umgebung abgegeben. Auf diese Weise wird verhindert, dass noch in der vergorenen Biomasse enthaltene Biogasreste während des Entladens eine Gefahr für das Bedienungspersonal darstellen.

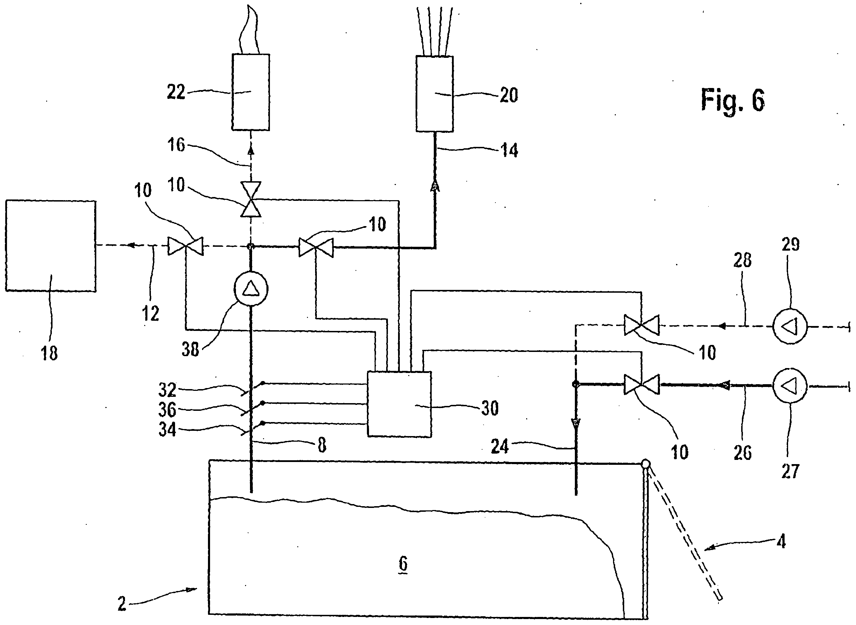

Ist der Fermenter 2 wieder mit frischer Biomasse beladen, wird die Be- und Entladeöffnung 4 geschlossen, die Verbindung zwischen Biogasauslass 8 und Abgaskamin 20 über die erste Biogas/Abgas-Leitung 14 wird aufrecht erhalten und die Steuereinrichtung 30 öffnet das Ventil 10 in der Abgasleitung 26, so das kohlendioxidhaltiges Abgas in den Fermenter 2 gepumpt wird - siehe Fig. 6. Dies wird solange fortgesetzt, bis die durch den zweiten Messfühler 34 erfasste Kohlendioxidkonzentration in dem Biogasauslass 8 einen zweiten Grenzwert erreicht bzw. übersteigt.

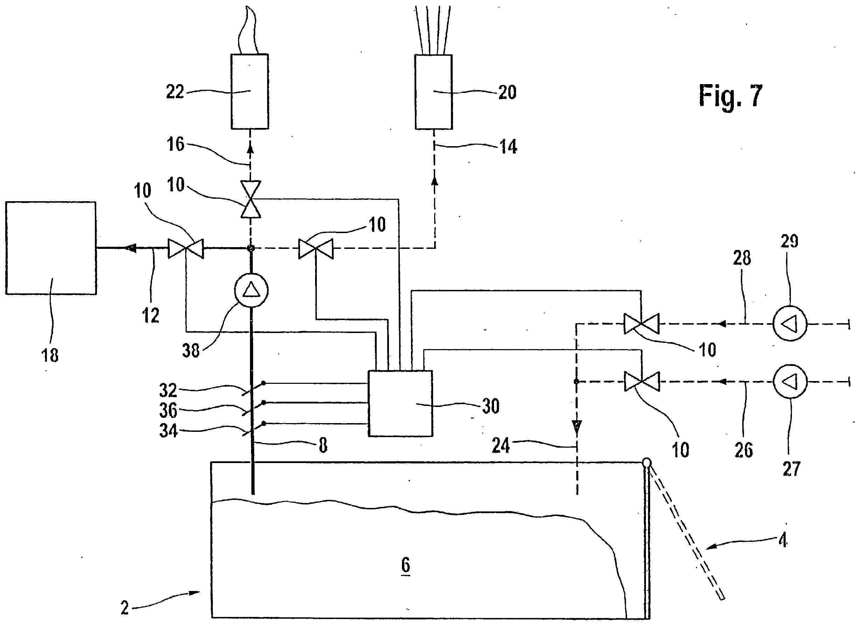

Ist dieser zweite Grenzwert für die Kohlendioxidkonzentration erreicht, wird durch die Steuereinrichtung 30 das Ventil 10 in der Abgasleitung 26 und in der ersten Biogas/Abgas-Leitung 14 geschlossen und das Ventil 10 in der Biogasleitung 12 geöffnet, wie dies in Fig. 7 dargestellt ist. Damit ist die Phase der Biogasproduktion wieder erreicht und das in dem Fermenter 2 erzeugte Biogas wird über die Biogasleitung 12 dem BHKW 18 zugeführt.

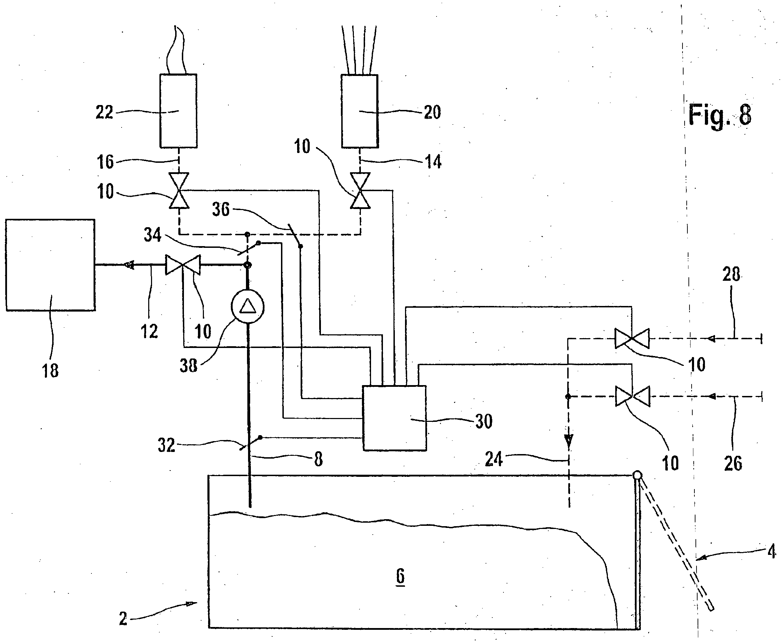

Bei der vorstehend beschriebenen Ausführungsform sind alle Messfühler 32, 34, 36 in dem Biogasauslass 8 angeordnet. Alternativ können der zweite und dritte Messfühler 24, 36 auch in der ersten bzw. zweiten Biogas/Abgas-Leitung 14, 16 angeordnet werden. Fig. 8 zeigt eine alternative Ausgestaltung der Erfindung, die sich von der Ausführungsform nach den Fig. 1 bis 7 dadurch unterscheidet, dass die erste und zweite Biogas/Abgas-Leitung 14, 16 zu einer gemeinsamen Biogas/Abgas-Leitung 40 zusammengefasst sind, bevor sie in den Biogasauslass 8 münden, Der zweite. Messfühler zur Erfassung der Kohlendioxidkonzentration ist in der gemeinsamen Biogas/Abgas-Leitung 40 angeordnet und der dritte Messfühler 36 ist in der ersten Biogas/Abgas-Leitung 14 angeordnet. Im übrigen stimmt diese zweite Ausführungsform der Erfindung mit der ersten Ausführungsform überein. Auch die Funktionsweise ist identisch.

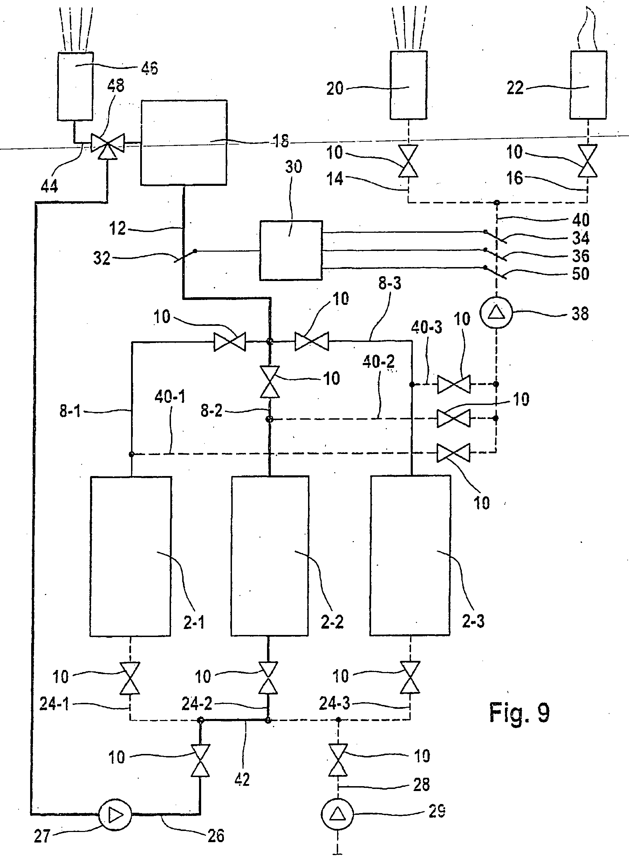

Die Figuren 9 bis 15 zeigen eine dritte Ausführungsform einer Biogasanlage gemäß der vorliegenden Erfindung, bei der drei Fermenter 2-1, 2-2 und 2-3 im Parallelbetrieb vorgesehen sind.. Einander entsprechende Komponenten sind mit den gleichen Bezugszeichen versehen. Bei der Biogasanlage nach den Figuren 9 bis 15 ist jeder der drei Fermenter 2-i mit einem Spülgaseinlass 24-1, 24-2 und 24-3 versehen, die jeweils mit einem Ventil 10 absperrbar sind. Die drei Spülgaseinlässe 24-i sind zu einem gemeinsamen Spülgaseinlass 42 zusammengefasst. In den gemeinsamen Spülgaseinlass 42 münden eine Abgasleitung 26 und eine Frischtuftleitung 28, die jeweils durch ein Ventil 10 absperrbar sind.

Jeder der drei Fermenter 2-i ist mit einem Biogasauslass 8-1, 8-2 und 8-3 versehen, der jeweils mit einem Ventil 10 absperrbar sind. Die erste Biogas/Abgas-Leitung 14 zu dem Abgaskamin 20 und die zweite Biogas/Abgas-Leitung 16 zu der Abgasfackel 22 sind zu einer gemeinsamen Biogas/Abgas-Leitung 40 zusammengefasst in der ein Gebläse 38 angeordnet ist. Nach dem Gebläse 38 spaltet sich die gemeinsame Biogas/Abgas-Leitung 40 auf in eine erste, eine zweite und eine dritte Teil-Biogas/Abgas-Leitung 40-1, 40-2 und 40-3. Die erste Teil-Biogas/Abgas-Leitung 40-1 mündet zwischen Ventil 10 und dem ersten Biofermenter 2-1 in den ersten Biogasauslass 8-1. Die zweite Teil-Biogas/Abgas-Leitung 40-2 mündet zwischen Ventil 10 und dem zweiten Biofermenter 2-2 in den zweiten Biogasauslass 8-2. Die dritte Teil-Biogas/Abgas-Leitung 40-3 mündet zwischen Ventil 10 und dem dritten Biofermenter 2-3 in den dritten Biogasauslass 8-3. Die drei Teil-Biogas/Abgas-Leitungen 40-1, 40-2 und 40-3 sind jeweils durch ein Ventil 10 absperrbar. Die drei Biogasauslässe 8-1. 8-2 und 8-3 münden in eine gemeinsame Biogasleitung 12, die zu einem Blockheizkraftwerk 18 führt. Eine Auspuffleitung 44 aus dem BHKW 18 mündet einen zweiten Abgaskamin 46. Die Abgasleitung 26 ist über ein 3-Wege-Ventil 48 mit der Auspuffleitung 44 verbunden, d. h. das in dem BHKW 18 anfallende kohlendioxidhaltige Abgas wird zum Spülen eines abzuschaltenden Fermenters 2-i verwendet. Durch das 3-Wege-Ventil kann der Volumenstrom des Abgases, der zum Spülen eines Fermenters 2-i über die Abgasleitung 26 geschickt wird und die Menge des Abgases, die über den zweiten Abgaskamin 46 an die Umgebung abgegeben wird, geregelt werden.

Ein erster Messfühler 32 ist zur Erfassung der Methankonzentration in der gemeinsamen Biogasleitung 12 angeordnet. Ein zweiter Messfühler 34 zur Erfassung der Kohlendioxidkonzentration, ein dritter Messfühler 36 zur Erfassung des Volumenstroms und vierter Messfühler 50 zur Erfassung der Methankonzentration ist in der gemeinsamen Biogas/Abgas-Leitung 40 in Strömungsrichtung nach dem Gebläse 38 angeordnet. Die vier Messfühler 32, 34, 36 und 50 sind mit einer Steuereinrichtung 30 verbunden. Ebenfalls mit der Steuereinrichtung verbunden sind die verschiedenen Ventile 10. Aus Gründen der Übersichtlichkeit sind diese Steuerleitungen nicht in den Figuren 9 bis 15 eingezeichnet.

In den Figuren 9 bis 15 wird das Abschalten und wieder Anfahren des zweiten Fermenters 2-2 dargestellt, wobei die Figuren 9 bis 15 die gleichen Phasen und Betriebszustände wie die Figuren 1 bis 7 darstellen. Die Biogasproduktion des ersten und dritten Fermenters 2-1 und 2-3 läuft während des Abschaltens und wieder Anfahrens des zweiten Fermenters 2-2 kontinuierlich durch.

Fig. 9 zeigt die erste Phase des Abschaltens des Fermenters 2-2 bei der kohlendioxidhaltiges Abgas aus dem BHKW 18 über das 3-Wege-Ventil 48 und die Abgasleitung 26, das Abgasgebläse 27 und den zweiten Spülgaseinlass 24-2 in das Innere des Fermenters 2-2 gepumpt wird. Der zweite Biogasauslass 8-2 ist nach wie vor mit der gemeinsamen Biogasleitung 12 verbunden, so dass das Biogas/Abgas-Gemisch weiter dem BHKW 18 zugeführt wird.

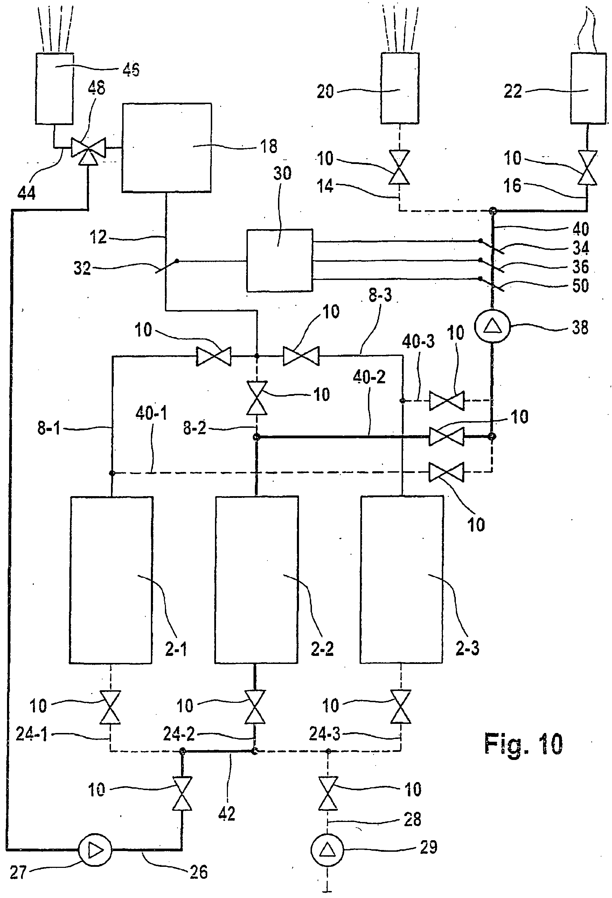

Erst wenn die durch den ersten Messfühler 32 in der gemeinsamen Biogasleitung 12 erfasste Methankonzentration unter einen oberen Grenzwert abgesunken ist, wird das Ventil 10 im zweiten Biogasauslass 8-2 durch die Steuereinrichtung 30 geschlossen und das Ventil 10 in der zweiten Teil-Biogas/Abgas-Leitung 40-2 und in der zweiten Biogas/Abgas-Leitung 16 wird geöffnet, wie dies in Fig. 10 dargestellt ist. In dieser zweiten Phase des Abschaltens des Fermenters 2-2 wird das Biogas/Abgas-Gemisch in der Abgasfackel 22 verbrannt. Gegebenenfalls kann dieser Verbrennungsprozess durch Zugabe von zusätzlichem Brennstoff unterstützt werden.

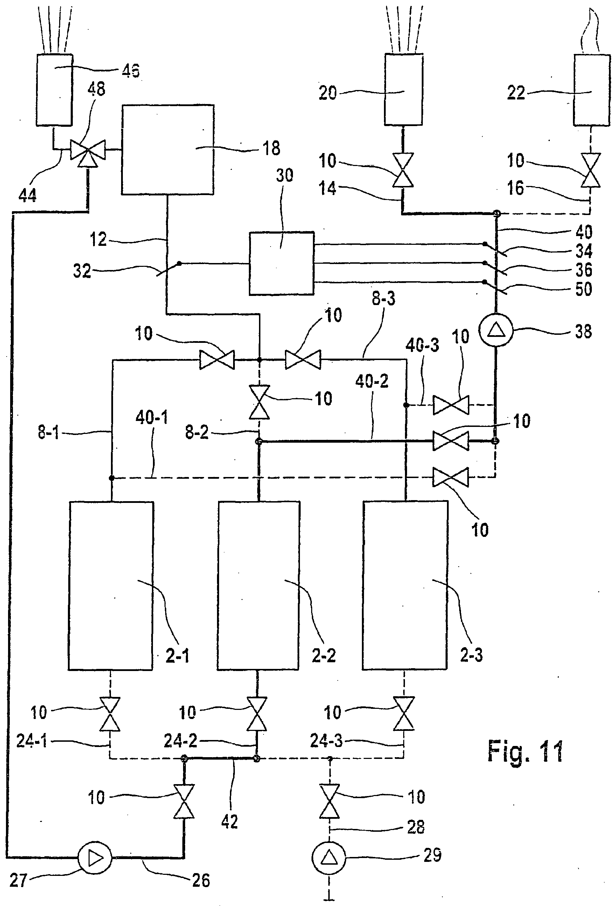

Wenn die durch den vierten Messfühler 50 in der gemeinsamen Biogas/Abgas-Leitung 40 erfasste Methankonzentration unter einen mittleren Grenzwert abgesunken ist, wird das Ventil 10 in der zweiten Biogas/Abgas-Leitung 16 durch die Steuereinrichtung 30 geschlossen und das Ventil 10 in der ersten Biogas/Abgas-Leitung 14 wird geöffnet, wie dies in Fig. 11 dargestellt ist. In dieser dritten Phase des Abschaltens des Fermenters 2-2 wird das Biogas/Abgas-Gemisch über den Abgaskamin 20 an die Umgebung abgegeben.

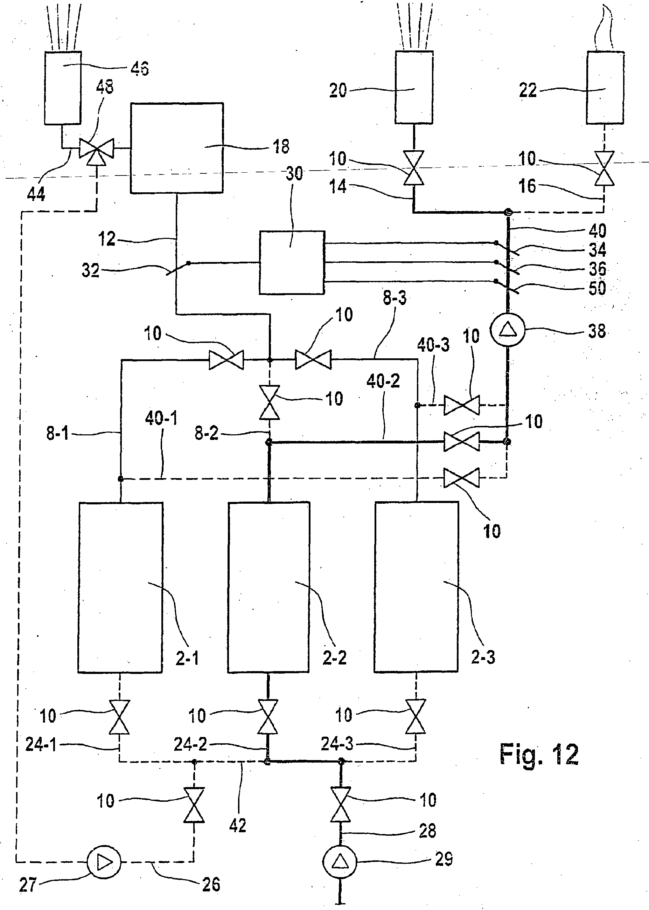

Wenn die durch den vierten Messfühler 50 in der gemeinsamen Biogas/Abgas-Leitung 40 erfasste Methankonzentration unter einen unteren Grenzwert abgesunken ist, wird das Ventil 10 in der Abgasleitung 26 durch die Steuereinrichtung 30 geschlossen, das 3-Wege-Ventil 48 wird entsprechend geschaltet und das Ventil 10 in der Frischluftleitung 28 wird geöffnet, wie dies in Fig. 12 dargestellt ist. In dieser vierten Phase des Abschaltens des Fermenters 2-2 wird über die Frischluftleitung 28 und den Spülgaseinlass 24 durch das Frischluftgebläse 29 Frischluft, in den Fermenter 2-2 gepumpt. Das Abgas/Luft-Gemisch wird weiter über den zweiten Biogasauslass 8-2, die zweite Teil-Biogas/Abgas-Leitung 40-2, die gemeinsame Biogas/Abgas-Leitung 40 und die erste Biogas/Abgas-Leitung 14 in dem Abgaskamin 20 an die Umgebung abgegeben. Gegebenenfalls kann dies durch das Gebläse 38 unterstützt werden.

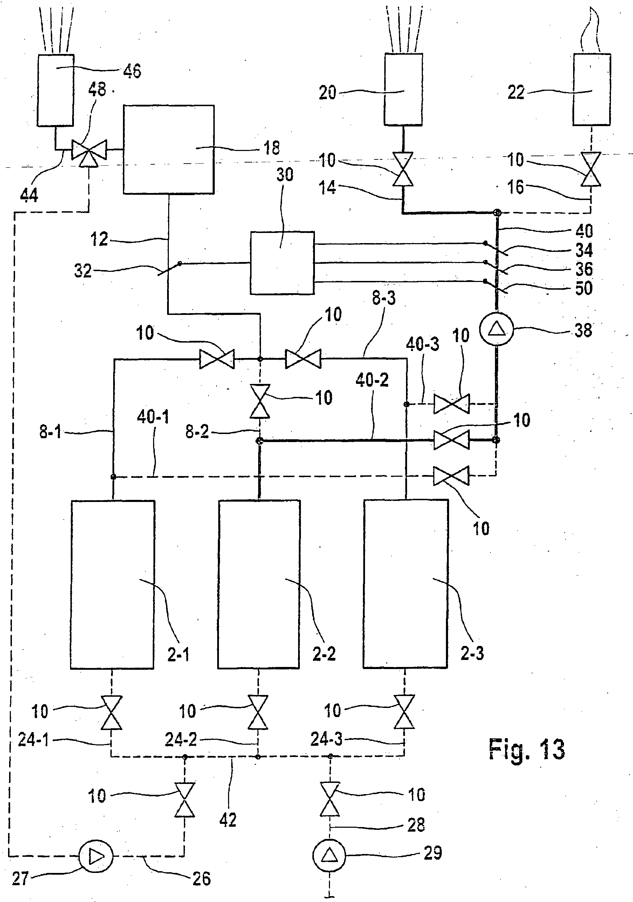

Wenn die durch den zweiten Messfühler 34 in der gemeinsamen Biogasleitung 40 erfasste Kohlendioxidkonzentration unter einen ersten Grenzwert abgesunken ist, wird das Ventil 10 in der Frischluftleitung 28 durch die Steuereinrichtung 30 geschlossen und das Frischluftgebläse 29 abgeschaltet, wie dies in Fig. 13 dargestellt ist. Die in den Figuren 9 bis 15 nicht dargestellte Be- und Entladeöffnung wird geöffnet. Gleichzeitig wird über das Gebläse 38 in die gemeinsamen Biogas/Abgas-Leitung 40 Frischluft über die offene Be- und Entladeöffnung angesaugt und über den Abgaskamin 20 an die Umgebung abgegeben. Auf diese Weise wird verhindert, dass noch in der vergorenen Biomasse enthaltene Biogasreste während des Entladens eine Gefahr für das Bedienungspersonal darstellen. Auch Abgase eines zum Be- und Entladen verwendeten Radladers werden damit abgesaugt.

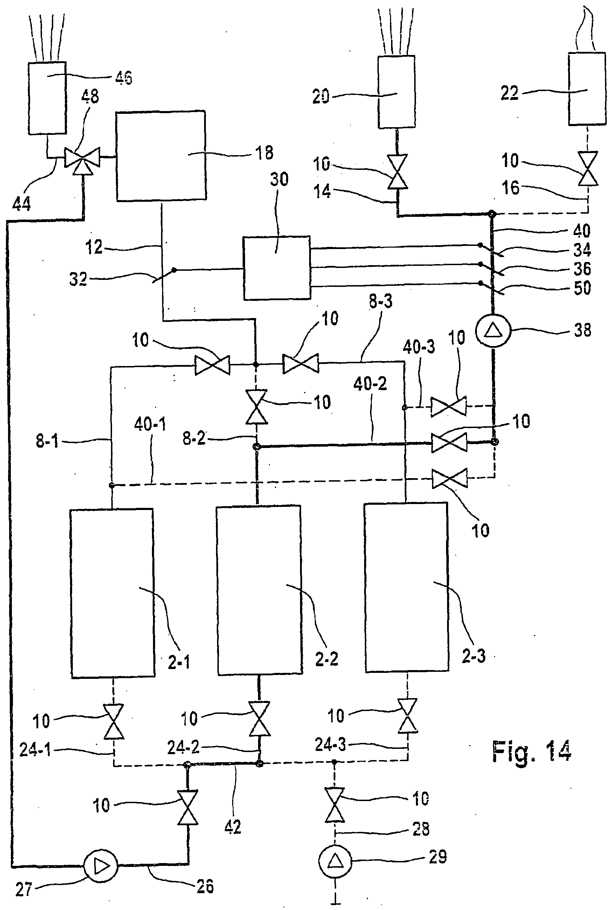

Ist der Fermenter 2-2 wieder mit frischer Biomasse beladen, wird die Be- und Entladeöffnung geschlossen, die Verbindung zwischen zweiten Biogasauslass 8-2 und Abgaskamin 20 über die zweite Teil-Biogas/Abgas-Leitung 40-2, die gemeinsame Biogas/Abgas-Leitung und die erste Biogas/Abgas-Leitung 14 wird aufrecht erhalten und die Steuereinrichtung 30 öffnet das Ventil 10 in der Abgasleitung 26 und schaltet das 3-Wege-Ventil 48 in der Auspuffleitung 44 des BHKW 18, so das kohlendioxidhaltiges Abgas in den Fermenter 2-2 gepumpt wird - siehe Fig. 14. Dies wird solange fortgesetzt, bis die durch den zweiten Messfühler 34 erfasste Kohlendioxidkonzentration in der gemeinsamen Biogas/Abgas-Leitung 40 einen zweiten Grenzwert erreicht bzw. übersteigt.

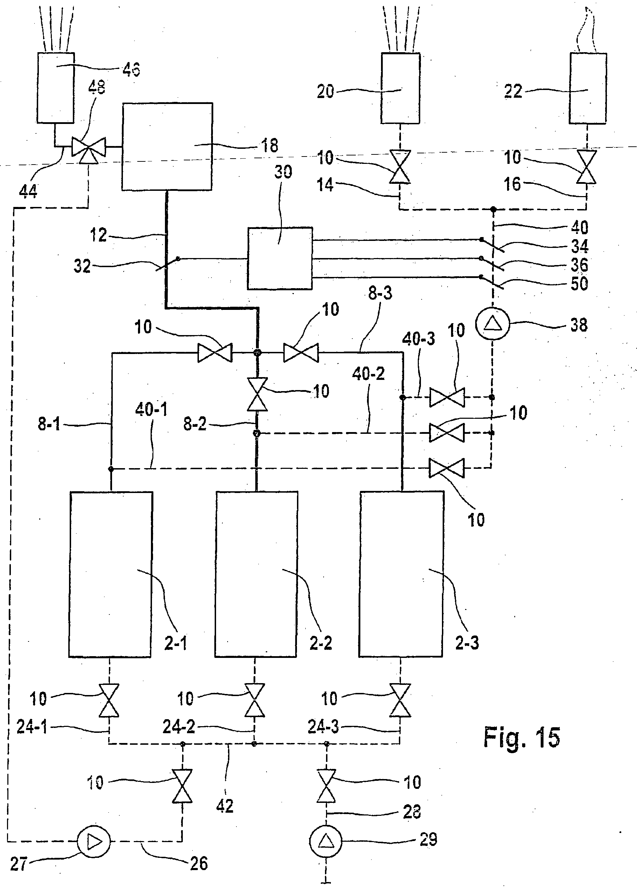

Ist dieser zweite Grenzwert für die Kohlendioxidkonzentration erreicht, wird durch die Steuereinrichtung 30 das Ventil 10 in der Abgasleitung 26 geschlossen, das 3-Wege-Ventil 38 geschalten, das Ventil 10 in der zweiten Teil-Biogas/Abgas-Leitung 40-2 wird geschlossen und das Ventil 10 in dem zweiten Biogasauslass 8-2 geöffnet, wie dies in Fig. 15 dargestellt ist. Damit hat auch der zweite Fermenter 2-2 wieder die Phase der Biogasproduktion erreicht und das in dem Fermenter 2-2 erzeugte Biogas wird über die Biogasleitung 12 dem BHKW 18 zugeführt. Die Zuschaltung des Biogasauslasses 8-2 zu der gemeinsamen Biogasleitung 12 erfolgt erst dann, wenn die durch den vierten Messfühler 50 erfasste Methankonzentration einen vierten Grenzwert erreicht. Dieser vierte Grenzwert stimmt mit dem oberen Grenzwert überein.

Das Ventil 10 in der Abgasleitung 26 kann entfallen, da dessen Funktion auch durch das 3-Wege-Ventil 48 übernommen werden kann.

Statt die Biogasleitung 12 direkt mit dem BHKW 18 zu verbinden, kann dem BHKW 18 vorzugsweise eine Gasaufbreitungseinrichtung (nicht dargestellt) vorgeschaltet werden. Durch die Gasaufbereitungseinrichtung wird mittels Druckwasserwäsche, Filtern oder Membranen die Qualität des erzeugten Biogases auf die Qualitätsstufe von Erdgas angehoben, d. h. es wird insbesondere der Methananteil erhöht und der Kohlendioxidanteil wird verringert.

Nachfolgend sind beispielhafte Zahlenwerte für die verschiedenen Grenzwerte angegeben:

| | | |
|---|---|---|
| Methankonzentration: | oberer Grenzwert | 30% bis 50% |
| | mittlerer Grenzwert | 10% bis 20% |
| | unterer Grenzwert | 0% bis 3% |
| | vierter Grenzwert | 30% bis 50% |
| | | |
| Kohlendioxidkonzentration: | erster Grenzwert | 0,5% bis 2% |
| | zweiter Grenzwert | 5% bis 15% |

Der Abgasvolumenstrom in der Abgasleitung 26 beträgt je nach Größe der Fermenter und der Menge des zur Verfügung stehenden Abgases zwischen 150 und 1000 m³/h. Der Frischluftvolumenstrom in der Frischluftleitung 28 beträgt zwischen 1000 und 5000 m³/h.

### Bezugszeichenliste

- 2: Fermenter
- 4: Be- und Entladeöffnung
- 6: Biomasse
- 8: Biogasauslass
- 10: Ventile
- 12: Biogasleitung
- 14: erste Biogas/Abgas-Leitung
- 16: zweite Biogas/Abgas-Leitung
- 18: Blockheizkraftwerk
- 20: Abgaskamin
- 22: Abgasfackel
- 24: Spülgaseinlass
- 26: Abgasleitung
- 27: Abgasgebläse
- 28: Frischluftleitung
- 29: Frischluftgebläse
- 30: Steuereinrichtung
- 32: erster Messfühler (Methankonzentration)
- 34: zweiter Messfühler (Kohlendioxidkonzentration)
- 36: dritter Messfühler (Volumenstrom)
- 38: Gebläse
- 40: gemeinsame Biogas/Abgas-Leitung
- 40-1: erste Teil-Biogas/Abgas-Leitung
- 40-2: zweite Teil-Biogas/Abgas-Leitung
- 40-3: dritte Teil-Biogas/Abgas-Leitung
- 42: gemeinsamer Spülgaseinlass
- 44: Auspuffleitung
- 46: zweiter Abgaskamin
- 48: 3-Wege-Ventil
- 50: vierter Messfühler (Methankonzentration)

## Patentansprüche

1. Biogasanlage zur Erzeugung von Biogas mit
wenigstens einem nach dem Prinzip der Trockenfermentation arbeitenden Fermenter (2) zur Erzeugung von Biogas im Batch-Betrieb mit einem Biogasauslass (8) und einem Spülgaseinlass (24);
einer Biogasleitung (12) die mit dem Biogasauslass (8) verbindbar ist;
einer Abgasleitung (26) mittels der kohlendioxidhaltiges Abgas dem Spülgaseinlass (24) zuführbar ist;
einem Abgaskamin (20), der über eine erste Biogas/Abgas-Leitung (14) mit dem Biogasauslass (8) verbindbar ist;
einer Abgasfackel (22), die über eine zweite Biogas/Abgas-Leitung (16) mit dem Biogasauslass (8) verbindbar ist;
einer Frischluftleitung (28), die mit dem Spülgaseinlass (24) verbindbar ist;
einer Steuereinrichtung (30) zur Verbindung des Biogasauslasses (8) mit der Biogasleitung (12) oder mit dem Bioabgaskamin (20) über die erste Biogas/Abgas-Leitung (14) oder mit der Abgasfackel (22) über die zweite Biogas/Abgas-Leitung (16) und zur Verbindung des Spülgaseinlasses (24) mit der Abgasleitung (26) oder mit der Frischluftleitung (28); und
einer Messeinrichtung (32, 34), die mit der Steuereinrichtung (30) verbunden ist und einen ersten Messfühler (32) zur Erfassung der Methankonzentration und einen zweiten Messfühler (34) zur Erfassung der Kohlendioxidkonzentration in dem aus dem wenigstens einen Fermenter (2) austretenden Gasgemisch umfasst.

2. Biogasanlage nach Anspruch 1, **dadurch gekennzeichnet, dass** die Messeinrichtung (32, 34) in dem Biogasauslass (8) angeordnet ist.

3. Biogasanlage nach Anspruch 1, **dadurch gekennzeichnet, dass** eine Mehrzahl von Fermentern (2-i) vorgesehen sind, deren Biogasauslässe (8-i) in die gemeinsamen Biogasleitung (12) münden und dass der erste Messfühler zur Erfassung der Methankonzentration in der gemeinsamen Biogasleitung angeordnet ist.

4. Biogasanlage nach Anspruch 3, **dadurch gekennzeichnet, dass** die Biogasauslässe (8-i) selektiv über eine gemeinsame Biogas/AbgasLeitung (40) mit dem Abgaskamin (20) oder der Abgasfackel (22) verbindbar sind und dass der zweite Messfühler zur Erfassung der Kohlendioxidkonzentration in der gemeinsamen Biogas/Abgas-Leitung angeordnet ist.

5. Biogasanlage nach einem der vorhergehenden Ansprüche, **dadurch gekennzeichnet, dass** die Abgasleitung (26) Abgas aus einer Verbrennungskraftmaschine zuführt.

6. Biogasanlage nach einem der vorhergehenden Ansprüche, **dadurch gekennzeichnet, dass** die Biogasleitung (12) die Verbindung zu einer Biogasverwertungseinrichtung herstellt, die kohlendioxidhaltiges Abgas erzeugt.

7. Biogasanlage nach Anspruch 5, **dadurch gekennzeichnet, dass** die Biogasverwertungseinrichtung ein Blockheizkraftwerk (18) umfasst.

8. Biogasanlage nach Anspruch 5, **dadurch gekennzeichnet, dass** die Biogasverwertungseinrichtung eine Brennstoffzelle umfasst.

9. Biogasanlage nach Anspruch 5, **dadurch gekennzeichnet, dass** die Biogasverwertungseinrichtung eine Gasaufbereitungseinrichtung (44) umfasst.

10. Verfahren zum Abschalten eines Fermenters in einer Biogasanlage nach einem der vorhergehenden Ansprüche mit den Verfahrensschritten:
a) Beibehalten der Verbindung zwischen Biogasauslass (8) und Biogasleitung (12);
b) Verbinden der Abgasleitung (26) mit dem Spülgaseinlass (24) des abzuschaltenden Fermenters (2);
c) Spülen des abzuschaltenden Fermenters (2) mit Abgas aus der Abgasleitung (26) bis die durch den ersten Messfühler (32) erfasste Methankonzentration auf einen oberen Grenzwert abgesunken ist;
d) Trennen der Biogasleitung (12) von dem Biogasauslass (8) des abzuschaltenden Fermenters (2);
e) Verbinden des Biogasauslasses (8) des abzuschaltenden Fermenters (2) mit der ersten Biogas/Abgas-Leitung (14) und Zufuhr des Abgas/Biogas-Gemisches zu dem Bioabgaskamin (20) bis die durch den ersten Messfühler (32) erfasste Methankonzentration auf einen unteren Grenzwert abgesunken ist;
f) Trennen der Abgasleitung (26) von dem Spülgaseinlass (24) des abzuschaltenden Fermenters (2);
g) Verbinden der Frischluftleitung (28) mit dem Spülgaseinlass (24) des abzuschaltenden Fermenters (2) und Zufuhr von Frischluft in den abzuschaltenden Fermenter (2) bis die durch den zweiten Messfühler (34) erfasste Kohlendioxidkonzentration auf einen ersten Grenzwert abgesunken ist; und
h) Öffnen der Be- und Entladeöffnung (4) des abgeschalteten Fermenters (2).

11. Verfahren nach Anspruch 10, **dadurch gekennzeichnet, dass** zwischen dem Verfahrensschritt d) und dem Verfahrensschritt e) folgende Verfahrensschritte ausgeführt werden
d1) Verbinden des Biogasauslasses (8) des abzuschaltenden Fermenters (2) mit der zweiten Biogas/Abgas-Leitung (16) und Zufuhr des AbgasBiogas-Gemisches zu der Abgasfackel (22) bis die durch den ersten Messfühler (32) erfasste Methankonzentration auf einen mittleren Grenzwert abgesunken ist, der zwischen dem oberen und dem unteren Grenzwert liegt; und
d2) Trennen des Biogasauslasses (8) des abzuschaltenden Fermenters (2) von der zweiten Biogas/Abgas-Leitung (16).

12. Verfahren nach Anspruch 10 oder 11, weiter **gekennzeichnet durch** die Verfahrensschritte:
i) Verbinden der Frischluftleitung (28) mit dem Spülgaseinlass (24) und/oder dem Biogasauslass (8); und
j) Zufuhr von Frischluft in den abgeschalteten Fermenter (2) über die Be- und Entladeöffnung (4) **durch** Absaugen über den Spülgaseinlass (24) und/oder den Biogasauslass (8) während des Ent- und Beladens des abgeschalteten Fermenters (2).

13. Verfahren nach Anspruch 12, **dadurch gekennzeichnet, dass** während des Verfahrensschrittes j) der Mengenstrom des über den Spülgaseinlass (24) und/oder den Biogasauslass (8) abgesaugten Luft/Biogas/Abgas-Gemisches durch die Steuereinrichtung (30) überwacht wird.

14. Verfahren zum Anfahren eines frisch mit Biomasse beladenen Fermenters (2) nach einem der vorhergehenden Ansprüche 1 bis 9 mit den Verfahrensschritten:
a) Schließen der Be- und Entladeöffnung (4);
b) Verbinden des Biogasauslasses (8) mit der ersten Biogas/AbgasLeitung (14);
c) Verbinden der Abgasleitung (26) mit dem Spülgaseinlass (24) des anzufahrenden Fermenters (2) und Zufuhr von Abgas zu dem anzufahrenden Fermenter (2) bis die durch den zweiten Messfühler (34) erfasste Kohlendioxidkonzentration einen zweiten Grenzwertwert erreicht;
d) Trennen der Abgasleitung (26) von dem Spülgaseinlass (24);
e) Trennen der ersten Biogas!Abgas-Leitung (14) von dem Biogasauslass (8);
f) Verbinden der Biogasleitung (12) mit dem Biogasauslass (8).

15. Verfahren nach Anspruch 14, **dadurch gekennzeichnet, dass** der Verfahrensschritt f) ausgeführt wird, wenn die durch den ersten oder vierten Messfühler (32; 50) erfasste Methankonzentration einen vierten Grenzwert überschreitet.

16. Verfahren nach Anspruch 15, **dadurch gekennzeichnet, dass** der vierte Grenzwert der Methankonzentration gleich dem oberen Grenzwert der Methankonzentration ist.

17. Verfahren nach einem der vorhergehenden Ansprüche 10 bis 16, **dadurch gekennzeichnet, dass** die Abgasleitung (26) mit dem Auspuff einer Verbrennungskraftmaschine verbunden wird.

18. Verfahren nach einem der vorhergehenden Ansprüche 10 bis 16, **dadurch gekennzeichnet, dass** die Abgasleitung (26) mit dem Auspuff einer kohlendioxidhaltiges Abgas erzeugenden Biogasaufbereitungsseinrichtung verbunden ist.

19. Verfahren nach einem der vorhergehenden Ansprüche 10 bis 16, **dadurch gekennzeichnet, dass** die Abgasleitung (26) mit dem Auspuff einer Brennstoffzelle verbunden ist.

## Claims

1. Biogas installation for production of biogas having
at least one fermenter (2) which operates on the principle of dry fermentation for production of biogas in the batch mode with a biogas outlet (8) and a purging gas inlet (24);
a biogas line (12) that can be connected to the biogas outlet (8);
an off-gas line (26) by means of which off-gas containing carbon dioxide can be supplied to the purging gas inlet (24);
an off-gas chimney (20) which can be connected to the biogas outlet (8) via a first biogas/off-gas line (14);
an off-gas flare (22) which can be connected via a second biogas/off-gas line (16) to the biogas outlet (8);
a fresh air line (28) which can be connected to the purging gas inlet (24);
a control device (30) for connection of the biogas outlet (8) to the biogas line (12) or to the bio off-gas chimney (20) via the first biogas/off-gas line (14) or to the off-gas flare (22) via the second biogas/off-gas line (16) and for connection of the purging gas inlet (24) to the off-gas line (26) or to the fresh air line (28); and
a measurement device (32, 34) which is connected to the control device (30) and has a first measurement sensor (32) for detection of the methane concentration and a second measurement sensor (34) for detection of the carbon dioxide concentration in the gas mixture emerging from the at least one fermenter (2).

2. Biogas installation according to Claim 1,
**characterised in that**
the measurement device (32, 34) is arranged in the biogas outlet (8).

3. Biogas installation according to Claim 1,
**characterised in that**
a plurality of fermenters (2-i) are provided, whose biogas outlets (8-i) open into the common biogas line (12), and **in that** the first measurement sensor for detection of the methane concentration is arranged in the common biogas line.

4. Biogas installation according to Claim 3,
**characterised in that**
the biogas outlets (8-i) can be connected selectively via a common biogas/off-gas line (40) to the off-gas chimney (20) or to the off-gas flare (22), and **in that** the second measurement sensor for detection of the carbon dioxide concentration is arranged in the common biogas/off-gas line.

5. Biogas installation according to one of the preceding claims,
**characterised in that**
the off-gas line (26) supplies exhaust gas from an internal combustion engine.

6. Biogas installation according to one of the preceding claims,
**characterised in that**
the biogas line (12) connects to a biogas utilisation device which produces off-gas containing carbon dioxide.

7. Biogas installation according to Claim 5,
**characterised in that**
the biogas utilisation device comprises a cogeneration system (18).

8. Biogas installation according to Claim 5,
**characterised in that**
the biogas utilisation device comprises a fuel cell.

9. Biogas installation according to Claim 5,
**characterised in that**
the biogas utilisation device comprises a gas processing device (44).

10. Method for shutting down a fermenter in a biogas installation according to one of the preceding claims, having the following method steps:
a) maintaining the connection between the biogas outlet (8) and the biogas line (12);
b) connecting the off-gas line (26) to the purging gas inlet (24) of the fermenter (2) to be shut down;
c) purging of the fermenter (2) to be shut down with off-gas from the off-gas line (26) until the methane concentration detected by the first measurement sensor (32) has fallen to an upper limit value;
d) disconnecting the biogas line (12) from the biogas outlet (8) of the fermenter (2) to be shut down;
e) connecting the biogas outlet (8) of the fermenter (2) to be shut down to the first biogas/off-gas line (14), and supply of the off-gas/biogas mixture to the off-gas chimney (20) until the methane concentration detected by the first measurement sensor (32) has fallen to a lower limit value;
f) disconnecting the off-gas line (26) from the purging gas inlet (24) of the fermenter (2) to be shut down;
g) connecting the fresh air line (28) to the purging gas inlet (24) of the fermenter (2) to be shut down, and supply of fresh air into the fermenter (2) to be shut down, until the carbon dioxide concentration detected by the second measurement sensor (34) has fallen to a first limit value; and
h) opening of the loading and unloading opening (4) of the fermenter (2) that has been shut down.

11. Method according to Claim 10,
**characterised in that**
the following method steps are carried out between method step d) and method step e):
d1) connecting the biogas outlet (8) of the fermenter (2) to be shut down to the second biogas/off-gas line (16), and supply of the off-gas/ biogas mixture to the off-gas flare (22) until the methane concentration detected by the first measurement sensor (32) has fallen to a medium limit value which is between the upper and the lower limit value; and
d2) disconnecting the biogas outlet (8) of the fermenter (2) to be shut down from the second biogas/off-gas line (16).

12. Method according to Claim 10 or 11, further
**characterised by**
the following method steps:
i) connecting the fresh air line (28) to the purging gas inlet (24) and/or to the biogas outlet (8); and
j) supplying fresh air into the fermenter (2) that has been shut down via the loading and unloading opening (4) by sucking out via the purging gas inlet (24) and/or the biogas outlet (8) while the fermenter (2) that has been shut down is being unloaded and loaded.

13. Method according to Claim 12,
**characterised in that**,
during method step j), the volume flow of the air/biogas/offgas mixture sucked out via the purging gas inlet (24) and/or the biogas outlet (8) is monitored by the control device (30).

14. Method for starting a fermenter (2) which has been freshly loaded with biomass, according to one of the preceding Claims 1 to 9, having the following method steps:
a) closing of the loading and unloading opening (4);
b) connecting the biogas outlet (8) to the first biogas/off-gas line (14);
c) connecting the off-gas line (26) to the purging gas inlet (24) of the fermenter (2) to be started and supply of off-gas to the fermenter (2) to be started until the carbon dioxide concentration detected by the second measurement sensor (34) reaches a second limit value;
d) disconnecting the off-gas line (26) from the purging gas inlet (24);
e) disconnecting the first biogas/off-gas line (14) from the biogas outlet (8);
f) connecting the biogas line (12) to the biogas outlet (8).

15. Method according to Claim 14,
**characterised in that**
method step f) is carried out when the methane concentration detected by the first or fourth measurement sensor (32; 50) exceeds a fourth limit value.

16. Method according to Claim 15,
**characterised in that**
the fourth limit value of the methane concentration is equal to the upper limit value of the methane concentration.

17. Method according to one of the preceding Claims 10 to 16,
**characterised in that**
the off-gas line (26) is connected to the exhaust pipe of an internal combustion engine.

18. Method according to one of the preceding Claims 10 to 16,
**characterised in that**
the off-gas line (26) is connected to the exhaust pipe of a biogas processing device which produces an off-gas containing carbon dioxide.

19. Method according to one of the preceding Claims 10 to 16,
**characterised in that**
the off-gas line (26) is connected to the exhaust of a fuel cell.

## Revendications

1. Installation biologique de production de biogaz comprenant:
au moins un fermenteur (2) travaillant selon le principe de la fermentation à sec pour produire du biogaz dans un fonctionnement discontinu, avec une évacuation de biogaz (8) et une admission de gaz de balayage (24);
une conduite à biogaz (12) susceptible d'être reliée à l'évacuation de biogaz (8);
une conduite à gaz d'échappement (26), au moyen de laquelle les gaz d'échappement contenant du dioxyde de carbone sont susceptibles d'être amenés à l'admission de gaz de balayage (24);
une cheminée à gaz d'échappement (20), susceptible d'être reliée à l'évacuation de biogaz (8) par l'intermédiaire d'une première conduite à biogaz/gaz d'échappement (14);
une torchère à gaz d'échappement (22), susceptible d'être reliée à l'évacuation de biogaz (8) par l'intermédiaire d'une deuxième conduite à biogaz/gaz d'échappement (16);
une conduite à air neuf (28), susceptible d'être reliée à l'admission de gaz de balayage (24);
un dispositif de commande (30), pour assurer la liaison de l'évacuation de biogaz (8) à la conduite à biogaz (12) ou à la cheminée à gaz d'échappement (20), par l'intermédiaire de la première conduite à biogaz/gaz d'échappement (14), ou à la torchère à gaz d'échappement (22), par l'intermédiaire de la deuxième conduite à biogaz/gaz d'échappement (16), et pour assurer la liaison de l'admission de gaz de balayage (24) à la conduite à gaz d'échappement (26), ou à la conduite à air neuf (28); et
un dispositif de mesure (32, 34), relié au dispositif de commande (30) et comprenant une première sonde de mesure (32) pour appréhender la concentration en méthane et une deuxième sonde de mesure (34) pour appréhender la concentration en dioxyde de carbone dans le mélange de gaz sortant du au moins un fermenteur (2).

2. Installation biologique de production de biogaz selon la revendication 1,
**caractérisée en ce que**
le dispositif de mesure (32, 34) est disposé dans l'évacuation de biogaz (8).

3. Installation biologique de production de biogaz selon la revendication 1,
**caractérisée en ce**
**qu'**une pluralité de fermenteurs (2-i) sont prévus, dont les évacuations de biogaz (8-i) débouchent dans la conduite à biogaz (12) commune, et en ce que la première sonde dé mesure prévue pour appréhender la concentration en méthane est disposée dans la conduite à biogaz commune.

4. Installation biologique de production de biogaz selon la revendication 3,
**caractérisée en ce que**
les évacuations de biogaz (8-i) sont susceptibles d'être reliées sélectivement à la cheminée à gaz d'échappement (20) ou à la torchère à gaz d'échappement (22) par l'intermédiaire d'une conduite à biogaz/gaz d'échappement (40) commune, et **en ce que** la deuxième sonde de mesure prévue pour appréhender la concentration en dioxyde de carbone est disposé dans la conduite à biogaz/gaz d'échappement commune.

5. Installation biologique de production de biogaz selon l'une des revendications précédentes,
**caractérisée en ce**
**que** la conduite à gaz d'échappement (26) amène des gaz d'échappement provenant d'un moteur à combustion.

6. Installation biologique de production de biogaz selon l'une des revendications précédentes,
**caractérisée en ce que**
la conduite à biogaz (12) établit la liaison à un dispositif de valorisation du biogaz, générant les gaz d'échappement contenant du dioxyde de carbone.

7. Installation biologique de production de biogaz selon la revendication 5,
**caractérisée en ce que**
le dispositif de valorisation du biogaz comprend une centrale de chauffage à bloc (18).

8. Installation biologique de production de biogaz selon la revendication 5,
**caractérisée en ce que**
le dispositif de valorisation du biogaz comprend une pile à combustible.

9. Installation biologique de production de biogaz selon la revendication 5,
**caractérisée en ce que**
le dispositif de valorisation du biogaz comprend un dispositif de traitement des gaz (44).

10. Procédé de mise à l'arrêt d'un fermenteur dans une installation de biogaz selon l'une des revendications précédentes, comprenant les étapes suivantes :
a) conservation de la liaison entre évacuation de biogaz (8) et conduite à biogaz (12) ;
b) liaison de la conduite à gaz d'échappement (26) à l'admission de gaz de balayage (24) du fermenteur (2) devant être mis à l'arrêt ;
c) rinçage du fermenteur (2) devant être mis à l'arrêt avec les gaz d'échappement émanant de la conduite à gaz d'échappement (26), jusqu'à ce que la concentration en méthane appréhendée par la première sonde de mesure (32) soit descendue à une valeur limite supérieure ;
d) séparation entre conduite à biogaz (12) et évacuation de biogaz (8) du fermenteur (2) devant être mis à l'arrêt;
e) liaison de l'évacuation de biogaz (8) du fermenteur (2) devant être mis à l'arrêt à la première conduite à biogaz/gaz d'échappement (14) et amenée du mélange gaz d'échappement/biogaz à la cheminée à gaz d'échappement (20), jusqu'à ce que la concentration en méthane appréhendée par la première sonde de mesure (32) soit descendue à une valeur limite inférieure;
f) séparation de la conduite à gaz d'échappement (26) vis à vis de l'admission de gaz de balayage (24) du fermenteur (2) devant être mis à l'arrêt;
g) liaison de la conduite à air neuf (28) à l'admission de gaz de balayage (24) du fermenteur (2) devant être mis à l'arrêt et amenée d'air neuf dans le fermenteur (2) devant être mis à l'arrêt, jusqu'à ce que la concentration en dioxyde de carbone appréhendée par la deuxième sonde de mesure (34) soit descendue à une remière valeur limite ; et
h) ouverture de l'ouverture de chargement et déchargement (4) du fermenteur (2) devant être mis à l'arrêt.

11. Procédé selon la revendication 10,
**caractérisé en ce que**
les étapes de procédé suivantes doivent être effectuées entre l'étape de procédé d) et l'étape de procédé e):
d1) liaison de l'évacuation de biogaz (8) du fermenteur (2) devant être mis à l'arrêt à la deuxième conduite à biogaz/gaz d'échappement (16) et amenée du mélange gaz d'échappement/biogaz à la torchère à gaz d'échappement (22), jusqu'à ce que la concentration en méthane appréhendée par la première sonde de mesure (32) soit descendue à une valeur limite moyenne, située entre la valeur limite supérieure et la valeur limite inférieure; et
d2) séparation entre l'évacuation de biogaz (8) du fermenteur (2) devant être mis à l'arrêt et la deuxième conduite à biogaz/gaz d'échappement (16).

12. Procédé selon la revendication 10 ou 11, **caractérisé en outre par** les étapes de procédé:
i) liaison de la conduite à air neuf (28) à l'admission de gaz de balayage (24) et/ou à l'évacuation de biogaz (8) et
j) amenée d'air neuf dans le fermenteur (2) mis à l'arrêt, par l'intermédiaire de l'ouverture de chargement et déchargement (4), par aspiration par l'admission de gaz de balayage (24) et/ou l'évacuation de biogaz (8), pendant le déchargement et chargement du fermenteur (2) mis à l'arrêt.

13. Procédé selon la revendication 12,
**caractérisé en ce que**,
pendant l'étape de procédé j), le débit-masse du mélange air/biogaz/gaz d'échappement aspiré par l'admission de gaz de balayage (24) et/ou l'évacuation de biogaz (8) est surveillé par le dispositif de commande (30).

14. Procédé de démarrage d'un fermenteur (2) fraichement chargé de biomasse selon l'une des revendications 1 à 9 précédentes, avec les étapes de procédé:
a) fermeture de l'ouverture de chargement et déchargement (4) ;
b) liaison de l'évacuation de biogaz (8) à la première conduite à biogaz/gaz d'échappement (14);
c) liaison de la conduite à gaz d'échappement (26) à l'admission de gaz de balayage (24) du fermenteur (2) devant être démarré, et amenée de gaz d'échappement au fermenteur (2) devant être démarré, jusqu'à ce que la concentration en dioxyde de carbone appréhendée par la deuxième sonde de mesure (34) ait atteint une deuxième valeur limite;
d) séparation de la conduite à gaz d'échappement (26) vis à vis de l'admission de gaz de balayage (24);
e) séparation de la première conduite à biogaz/gaz d'échappement (14) vis à vis de l'évacuation de biogaz (8) ;
f) liaison de la conduite à biogaz (12) à l'évacuation de biogaz (8).

15. Procédé selon la revendication 14,
**caractérisé en ce que**
l'étape de procédé est effectuée lorsque la concentration en méthane, appréhendée par la première ou la quatrième sonde de mesure (32 ; 50), dépasse une quatrième valeur limité.

16. Procédé selon la revendication 15,
**caractérisé en ce que**
la quatrième valeur limite de concentration en méthane est égale à la valeur limite supérieure de concentration en méthane.

17. Procédé selon l'une des revendications 10 à 16 précédentes,
**caractérisé en ce que**
la conduite à gaz d'échappement (26) est reliée à l'échappement d'un moteur à combustion.

18. Procédé selon l'une des revendications 10 à 16 précédentes,
**caractérisé en ce que**
la conduite à gaz d'échappement (26) est reliée à l'échappement d'un dispositif de traitement du biogaz générant des gaz d'échappement contenant du dioxyde de carbone.

19. Procédé selon l'une des revendications 10 à 16 précédentes,
**caractérisé en ce que**
la conduite à gaz d'échappement (26) est reliée à l'échappement d'une pile à combustible.
